(19) **Europäisches Patentamt / European Patent Office / Office européen des brevets**

(11) **EP 4 233 900 A1**

(12) **EUROPEAN PATENT APPLICATION**
published in accordance with Art. 153(4) EPC

(43) Date of publication:
30.08.2023 Bulletin 2023/35

(21) Application number: 21887017.8

(22) Date of filing: 14.10.2021

(51) International Patent Classification (IPC):
A61K 39/35 (2006.01)    A61K 39/36 (2006.01)
A61K 39/29 (2006.01)    C07K 14/02 (2006.01)
A61K 38/17 (2006.01)    A61P 37/08 (2006.01)

(86) International application number:
PCT/RU2021/000437

(87) International publication number:
WO 2022/093065 (05.05.2022 Gazette 2022/18)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 26.10.2020 RU 2020135032

(71) Applicant: National Research Center Institute of
Immunology
Federal Medical-Biological Agency of Russia
Moscow, 115522 (RU)

(72) Inventors:
• KHAITOV, Musa Rakhimovich
  Moscow, 123056 (RU)
• VALENTA, Rudolf
  2604 Theresienfeld (AT)
• WEBER, Milena
  1190 Vienna (AT)
• CAMPANA, Raffaela
  1180 Vienna (AT)
• SHILOVSKIY, Igor Petrovich
  Vidnoe, 142701 (RU)

• SMIRNOV, Valerii Valerievich
  Dolgoprudny, 141700 (RU)
• ZHERNOV, Yurii Vladimirovich
  Moscow, 123242 (RU)
• ANDREEV, Sergei Mihailovich
  Moscow, 117519 (RU)
• SHATILOV, Artem Andreevich
  Moscow, 124498 (RU)
• TIMOFEEVA, Anastasiya Vitalievna
  Moscow, 124498 (RU)
• ILIINA, Nataliya Ivanovna
  Moscow, 115230 (RU)
• FEDENKO, Elena Sergeevna
  Moscow, p. Vnukovskoe, 108850 (RU)
• ELISYUTINA, Olga Gurievna
  Moscow, 115547 (RU)
• TRUKHIN, Victor Pavlovich
  Leningradskaya obl.,188538 (RU)
• YUDIN, Sergej Mikhajlovich
  Moscow, 123222 (RU)
• SKVORTSOVA, Veronika Igorevna
  Moscow, 117418 (RU)

(74) Representative: Gevers & Orès
Immeuble le Palatin 2
3 Cours du Triangle
CS 80165
92939 Paris La Défense Cedex (FR)

(54) **RECOMBINANT POLYPEPTIDE BASED ON BIRCH POLLEN ALLERGEN AND APPLE ALLERGEN**

(57) The invention relates to medicine, namely to biotechnology, immunology, and allergology, and concerns the production of a recombinant polypeptide for allergen-specific immunotherapy capable of inducing IgG-blocking antibodies to birch pollen allergen and to cross-food allergens. The recombinant polypeptide for the treatment or prevention of allergy to birch pollen and apple allergen comprises peptide fragments of wild-type allergens of birch pollen and apple linked to a surface PreS polypeptide of hepatitis B virus and has an amino acid sequence of SEO ID NO: 2. A recombinant polypeptide is produced that comprises epitopes necessary to activate protective antibodies against Bet v 1 and Mal d 1 in a single protein, which makes it possible to produce a single-component vaccine, instead of using vaccines comprising two or more allergen derivatives.

A-PreS

| A-P1 | A-P1 | PreS | A-P2 | A-P2 |

B-PreS

| B-P1 | B-P1 | PreS | B-P2 | B-P2 |

AB-PreS

| B-P1 | B-P1 | A-P1 | PreS | A-P2 | B-P2 | B-P2 |

# FIG. 2

## Description

### TECHNICAL FIELD

The invention relates to medicine, in particular to biotechnology, immunology and allergology, and concerns the production of a recombinant polypeptide for allergen-specific immunotherapy, which is capable of inducing blocking IgG antibodies to birch pollen allergen and cross-food allergens.

### BACKGROUND

Induction of allergen-specific IgG antibodies which block the binding of IgE antibodies to a sensitizing agent in patients is the main mechanism in allergen-specific immunotherapy (AIT) [1]. Allergen-specific blocking IgG antibodies induced during AIT reduce allergy symptoms by blocking the cross-linking of IgE antibodies on mast cells and basophils [2]. In addition, allergen-specific IgG antibodies induced during AIT can inhibit IgE-mediated presentation of allergen to T cells and thereby reduce the late phase of the allergic response [3]. Finally, allergen-specific IgG antibodies can bind an allergen, preventing an increase in allergen-specific IgE response in allergic patients exposed to allergen. Presumably, this mechanism leads to a long-term reduction of allergen-specific IgE antibodies in patients undergoing long-term AIT [4].

Allergy to birch pollen is common among people in Russia and other northern countries. It is often associated with oral allergy syndrome (OAS) and occurs when eating food of plant origin because IgE antibodies specific for the major birch pollen allergen Bet v 1 also cross-react with structurally related allergens of other plants [5]. In particular, such Bet v 1 cross-reactivity can be seen with apple allergen Mal d 1, which results in OAS in birch-allergic patients when eating apples. Allergen-specific immunotherapy with extracts comprising Bet v 1 or recombinant Bet v 1 derivatives induces not only protective IgG antibodies to Bet v 1, but also blocking IgG antibodies to apple allergen Mal d 1 [6]. For this reason, AIT aimed at treating birch pollen allergy partially protects from OAS, but this protection is not stable [7]. Therefore, there is a need to develop vaccines for AIT capable of inducing blocking IgG antibodies not only to the birch pollen allergen Bet v 1, but also to cross-food allergens.

A pharmaceutical composition has been developed for specific immunotherapy of allergies initiated by the major birch pollen allergen Bet v 1, the pharmaceutical composition comprises the hypoallergenic major birch pollen allergen Bet v 1 and/or pharmaceutically acceptable derivatives thereof. The hypoallergenic major birch pollen allergens are characterized by the lack of or a reduction in the binding of immunoglobulin-E, while maintaining therapeutically relevant T-cell stimulation (WO 03/072601 (MERK PATENT GMBH), 04.09.2003).

Recombinant allergens Bet v 1 are known which, when used, allow the specific IgE-binding capability to be reduced compared to the IgE-binding capability of naturally occurring allergen. The recombinant allergen Bet v 1 is a mutant of the naturally occurring allergen Bet v 1, wherein the mutant allergen Bet v 1 has at least four mutations, wherein each mutation is a substitution of one surface-exposed amino acid residue with another residue, which does not occur in the same position in the amino acid sequence of any known homologous protein within the taxonomic species (WO 02/40676 (ALC-ABELLO), 23.05.2002).

According to WO 2013/017591 (LOFARMA SPA, February 7, 2013) a hybrid protein was prepared for use in immunotherapy of patients allergic to pollen of *Malus domectica* and/or *Betula verrucosa*, the hybrid protein comprising a protein, which is a hypoallergenic mutant of the major allergen of *Malus domectica,* and a protein, which is a hypoallergenic mutant of the Bet v 1 major allergen of *Betula verrucosa* pollen, separated by a linker. This technology for creating hypoallergenic vaccines is characterized by site-directed mutations introduced into the DNA sequence of allergens. As a result, the polypeptide sequence of the allergen also changes, leading to a reduction in allergenicity. This approach allowed the inventors to reduce the IgE reactivity of the chimeric mutant protein. A disadvantage of the obtained chimeric mutant protein is its residual IgE reactivity. In addition, it comprises T-cell epitopes, i.e. there is a possibility of activating T cells that produce proinflammatory factors, which in turn reduces the safety of this protein.

Currently, a new generation of molecular AIT vaccines has been created allowing the IgG antibodies to occupy IgE antibody-binding sites on allergens [8].

It was shown that the occupation of IgE antibody-binding sites on allergens by IgG antibodies was achieved by fusing non-allergenic peptides derived from allergen-IgE binding sites with the hepatitis B virus PreS protein acting as a carrier. This new type of molecular allergy vaccines has many advantages over traditional allergen extract-based vaccines in terms of the method of their production, safety, and ease of clinical use [9]. Technology based on PreS carrier protein is also used to create hypoallergenic vaccines for the treatment of patients with birch pollen allergy [10]. In particular, in immunized rabbits, one of the candidates, named 2PAPB-PreS, also referred to as B-Pres, induced protective IgG antibodies better than the native recombinant allergen rBet v 1 [10].

The production of a hypoallergenic protein is known that consists of one allergen-derived hypoallergenic molecule fused to a second pathogen-derived non-allergenic protein. The allergen is selected from the group consisting of major birch pollen allergens, in particular Bet v 1 and Bet v 4, major timothy grass pollen allergens, major house dust mite allergens, major cat allergens Fel d 1 and Fel d 2, major bee allergens, major wasp allergens, and Ambrosia allergens (WO 2007/140505 (BIOMAY AG), 13.12.2007).

The closest prior art to the invention is a vaccine composition for the treatment or prevention of allergy to birch pollen allergen Bet v 1, comprising an effective amount of the allergen, in particular the birch pollen allergen Bet v 1, and a carrier protein, which is a hepatitis B PreS polypeptide (WO 2012/168487 (BIOMAY AG), 13.12.2012). The allergen-specific IgG antibodies induced by immunization with selected allergen-derived peptide fragments fused to a hepatitis B PreS polypeptide were found to be better target allergen IgE epitopes, while the immunization with wild-type allergen induces IgG against all parts of the allergen, including those which are not IgE-reactive.

However, this vaccine composition is not capable of inducing blocking IgG antibodies to cross-food allergens.

The object underlying the present invention is to provide an allergen-specific immunotherapy vaccine capable of inducing blocking IgG antibodies to a birch pollen allergen and to cross-food allergens, in particular to apple allergen Mal d 1.

The problem is solved by providing a recombinant polypeptide for the treatment or prevention of allergy to a birch pollen allergen and an apple allergen, comprising peptide fragments from wild-type allergens of birch pollen and apple, which are connected via a surface PreS polypeptide of hepatitis B virus, and having an amino acid sequence of SEQ ID NO: 2.

The recombinant polypeptide comprises at least four peptide fragments of wild-type birch pollen allergen Bet v 1 and at least two peptide fragments of wild-type apple allergen Mal d 1.

The wild-type allergen is selected from the group consisting of major birch pollen allergens, in particular Bet v 1, wherein two peptide fragments have an amino acid sequence of SEQ ID NO: 5, and the other two peptide fragments have an amino acid sequence of SEQ ID NO: 6.

In addition, the wild-type allergen is selected from the group consisting of major apple allergens, in particular Mal d 1, wherein one of the peptide fragments has an amino acid sequence of SEQ ID NO: 3, and another has an amino acid sequence of SEQ ID NO: 4.

The vaccine composition for the treatment or prevention of allergy to birch pollen and apple allergen comprises an effective amount of the recombinant polypeptide having an amino acid sequence of SEQ ID NO: 2 and a pharmaceutically acceptable excipient. The composition additionally comprises at least one adjuvant and/or preservative. The adjuvant is aluminum hydroxide.

The technical result of the claimed invention is the production of a recombinant polypeptide AB-PreS (SEQ ID NO: 2), that comprises epitopes necessary for activation of protective antibodies against Bet v 1 and Mal d 1 in a single peptide, which makes it possible to produce a single-component vaccine, instead of using vaccines comprising two or more allergen derivatives.

The resulting recombinant polypeptide AB-PreS, which comprises peptides from apple and birch allergen fused with PreS protein, completely lacks IgE reactivity, which is indicative of a greater safety of said protein. In addition, AB-PreS comprises apple and birch allergen peptides which mimic B-, but not T-cell epitopes of these allergens. The elimination of T-cell epitopes from the composition of AB-PreS makes it possible to achieve a more significant safety of the construct because of avoiding the possibility of activating T-cells which produce proinflammatory factors.

According to the present invention, the resulting recombinant polypeptide AB-PreS (SEQ ID NO: 2) comprises, in addition to peptide derivatives of wild-type birch pollen allergen Bet v 1 (SEO ID NO: 5 and SEO ID NO: 6), peptide derivatives of apple allergen Mal d 1 (SEO ID NO: 3 and SEQ ID NO: 4) between the birch allergen peptides and a surface PreS polypeptide of hepatitis B virus (SEQ ID NO: 7), which allows said recombinant construct to induce an increased immune response against allergenic molecules, and induce blocking IgG antibodies against the birch pollen allergen Bet v 1 and the apple allergen Mal d 1.

Thus, the resulting recombinant protein represented by SEQ ID NO: 2 demonstrates much more marked inhibition of IgE binding to Mal d 1 than the inhibition with the same doses of polypeptide B-PreS. As a result, the birch pollen allergy vaccine based on polypeptide AB-PreS induces a higher level of projective antibodies blocking IgE binding to Bet v 1 and Mal d 1 in comparison with the other tested vaccines.

**BRIEF DESCRIPTION OF GRAPHIC MATERIALS/FIGURES**

Fig. 1 shows multiple alignment of sequences of the allergen Bet v 1 and similar allergens.
The amino acid sequence of Bet v 1 was co-aligned with related allergens from fruits, vegetables, and pollen. Identical amino acids are indicated by dots, spaces are indicated by dashes, and the number of amino acids is given on the right side of the field.
Fig. 2 is a schematic representation of PreS protein-based polypeptides.
Construct A-PreS consists of two copies of each of two Mal d 1-derived peptides (A-P1 and A-P2) fused with PreS protein. Construct B-PreS consists of two copies of each of the two Bet v 1-derived peptides (B-P1 and B-P2) fused with PreS protein. Construct AB-PreS is similar in overall construction to B-PreS consisting of each of two Mal d 1-derived peptides arranged between sequences of PreS protein and birch allergen-derived peptides.

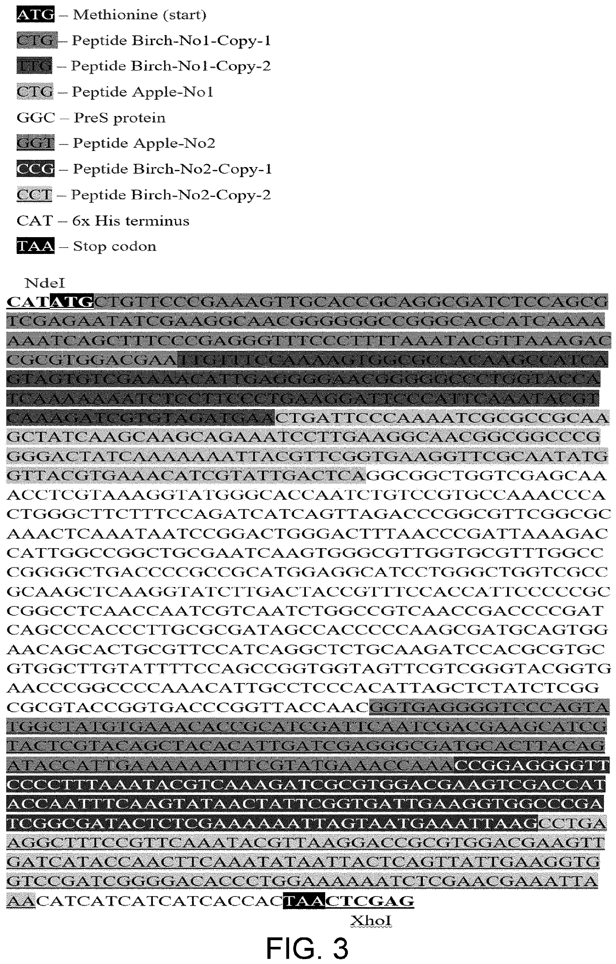

Fig. 3 shows the DNA sequence encoding polypeptide AB-PreS and a schematic representation of the regions encoding the main elements of the polypeptide.

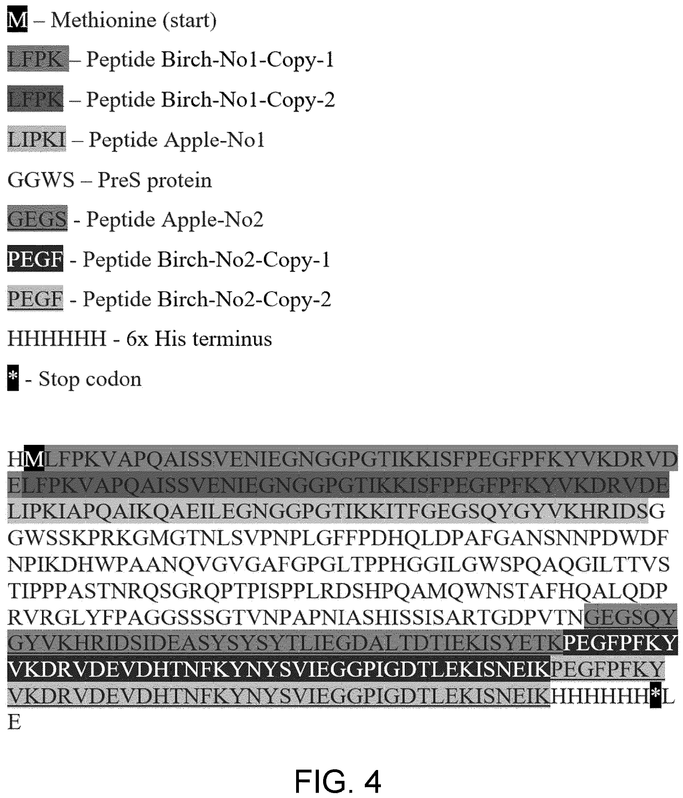

Fig. 4 shows the amino acid sequence of polypeptide AB-PreS and a schematic representation of its main elements.

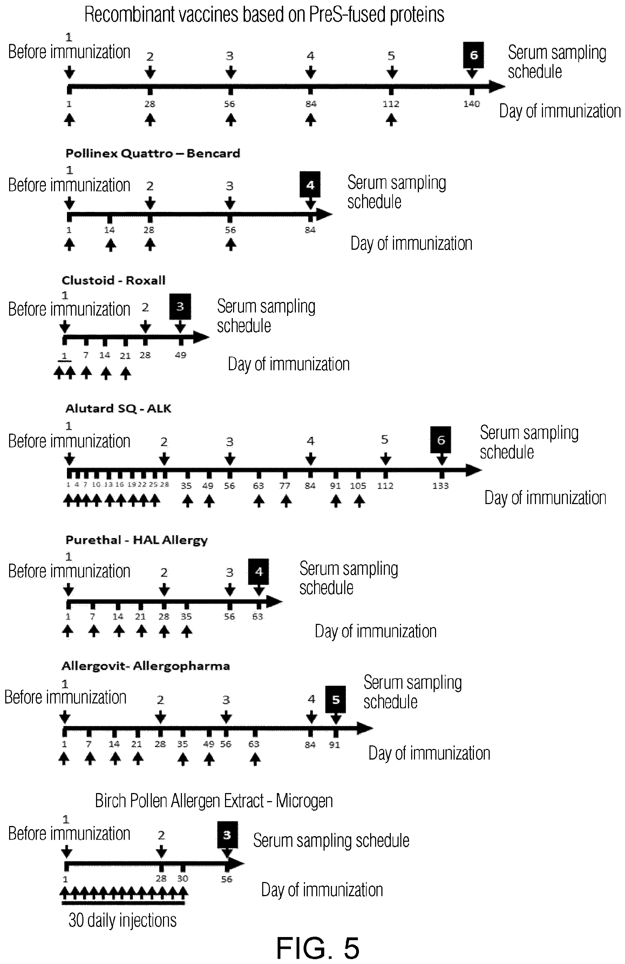

Fig. 5 is the protocol of immunization of rabbits with PreS protein-based recombinant vaccines and commercial allergen extract-based vaccines.

The final serum sampling is marked with a red square.

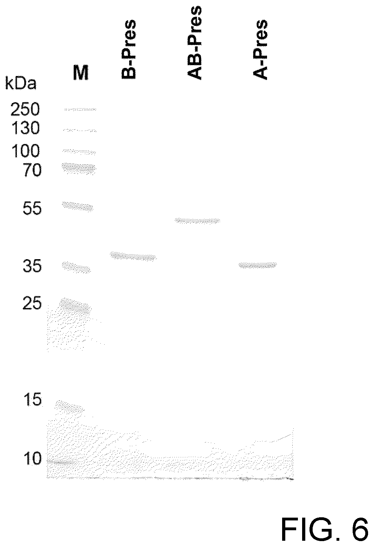

Fig. 6 is characteristic of recombinant proteins.

Coomassie-stained SDS-PAGE of purified PreS proteins (B-PreS, A-PreS and AB-PreS) and a molecular weight marker (M).

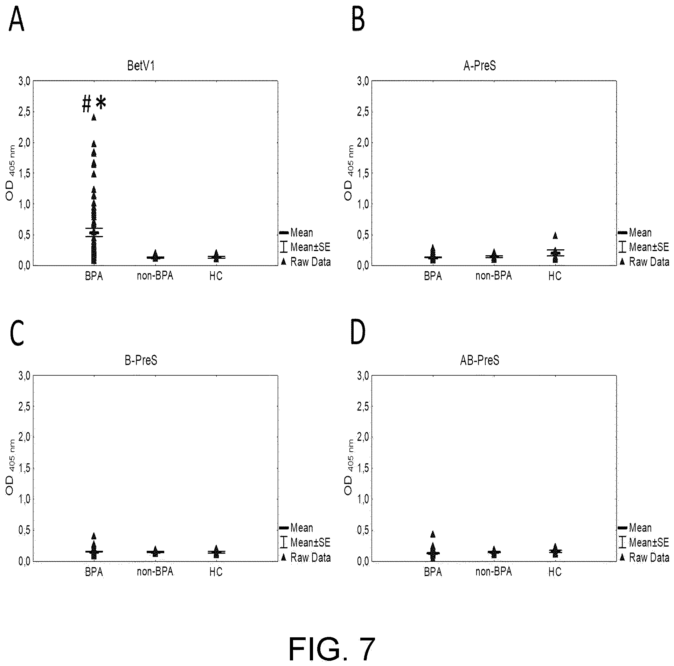

Fig. 7 shows the IgE-binding capacity of PreS proteins.

A recombinant Bet v 1 (A) and PreS proteins, namely A-PreS (B), B-Pres (C), and AB-PreS (D), were absorbed on an ELISA plate with further incubation with sera of patients with birch pollen allergy (BPA), without birch pollen allergy (non-BPA), and healthy volunteers (HVs) as a control.

Data are presented as mean optical density (OD) at 405 nm $\pm$ standard error. Statistical analysis performed with the Statistica 8.0 program using the Mann-Whitney U-test.

      * - statistically significantly different from "HVs".
      # - statistically significant compared to "non-BPA".

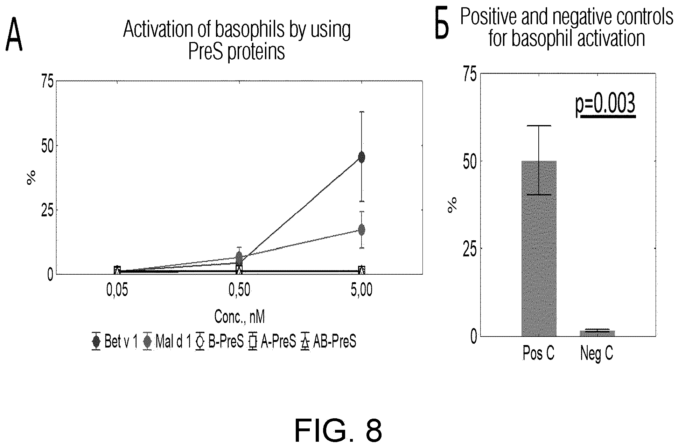

FIG. 8 is a comparison of A-PreS, B-PreS and AB-PreS with rBet v 1 and rMal d 1 for their ability to activate blood basophils.

The data shown are the percentages of activated basophils in the blood of four patients with birch pollen allergy (B1-B4) after stimulation with various concentrations (0.05-5 Nm) of Bet v 1 and Mal d 1, equimolar concentrations of B-PreS, A-PreS and AB-PreS (A). Anti-IgE antibodies were used as a positive control (Pos C), and the buffer solution was used as a negative control (Neg C) (B) .

Data are presented as the mean percentage of activated basophils $\pm$ standard error (N = 4).

Statistical analysis was performed with the Statistica 8.0 program using the Mann-Whitney U-test.

      * - statistically significantly different from "Neg C"
      x - statistically significantly different from "Bet v 1" and "Mal d 1".

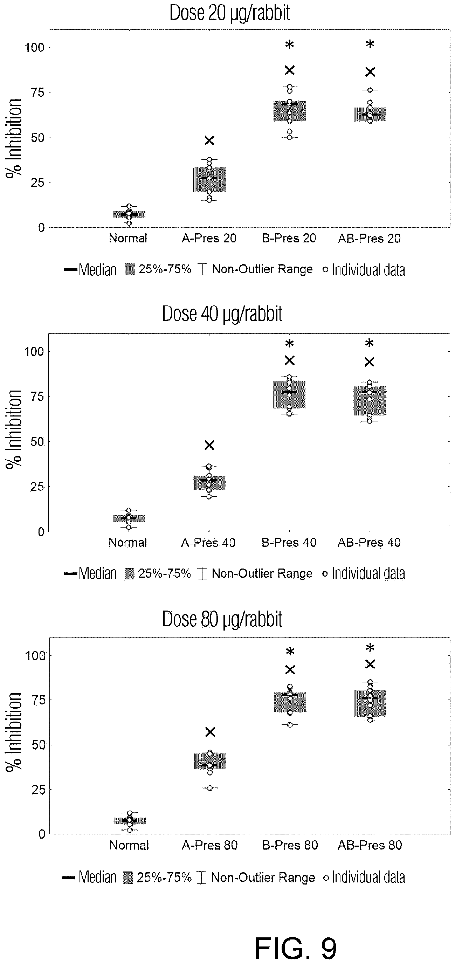

FIG. 9 shows the inhibition of the binding of patients' IgE antibodies to allergen Bet v 1, using the serum of rabbits immunized with various doses of PreS protein-based recombinant vaccines.

The percentage inhibition of IgE binding to rBet v 1 was determined for sera from 10 patients with birch pollen allergy. Pre-incubation of Bet v 1 with PBS was taken as 0% inhibition. Data are displayed as charts, for which 50% of the values are inside the rectangular shape; horizontal lines denote median values. Shown significant differences in inhibition were calculated using Student's t-test.

      x - statistically significant compared with the serum of non-immunized "Normal" rabbits.
      * - statistically significant compared with the serum of rabbits immunized with A-PreS.

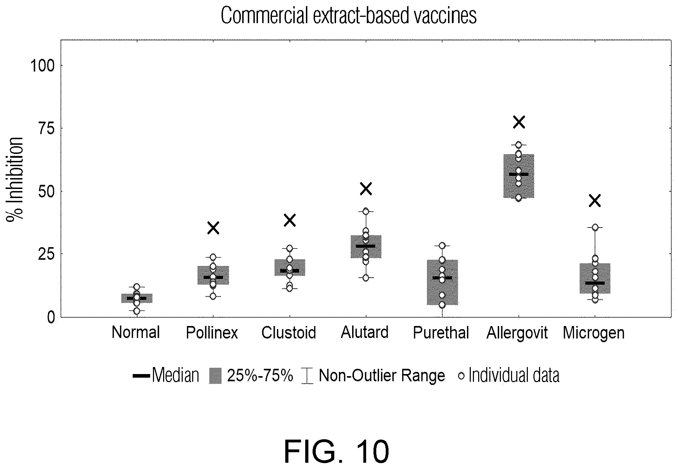

FIG. 10. shows the inhibition of IgE binding to Bet v 1, using sera from rabbits immunized with commercial allergen extract-based vaccines, one month after the last immunization.

The percentage inhibition of IgE binding to rBet v 1 was determined for sera from 10 patients with birch pollen allergy. Pre-incubation of Bet v 1 with PBS was taken as 0% inhibition. Data are plotted with 50% of the values within the rectangular box; horizontal lines denote median values. The shown significant differences in inhibition were calculated using Student's t-test.

$\times$ - statistically significant compared with sera from non-immunized "Normal" rabbits.

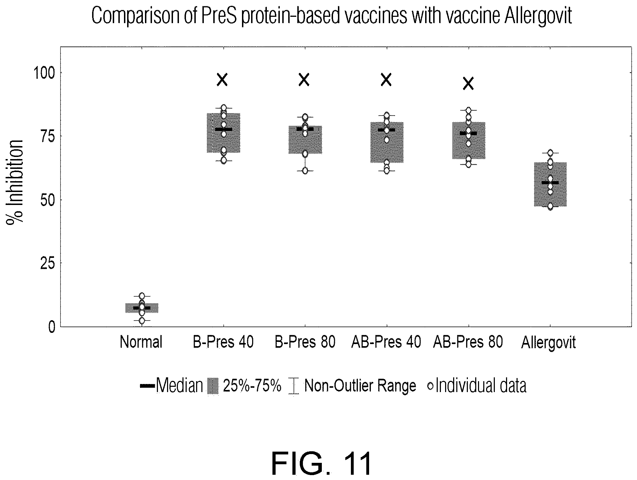

Fig. 11. shows the inhibition of the binding of patients' IgE antibodies to Bet v 1, using sera from rabbits immunized with recombinant PreS protein-based vaccines, in comparison with Allergovit.

The percentage inhibition of IgE binding to rBet v 1 was determined for sera from 10 patients with birch pollen allergy. Preincubation of Bet v 1 with PBS was taken as 0% inhibition. Data are plotted with 50% of the values within the rectangular box; horizontal lines denote median values. The shown significant differences in inhibition were calculated using Student's t-test.

x - statistically significant compared with sera from rabbits immunized with "Allergovit".

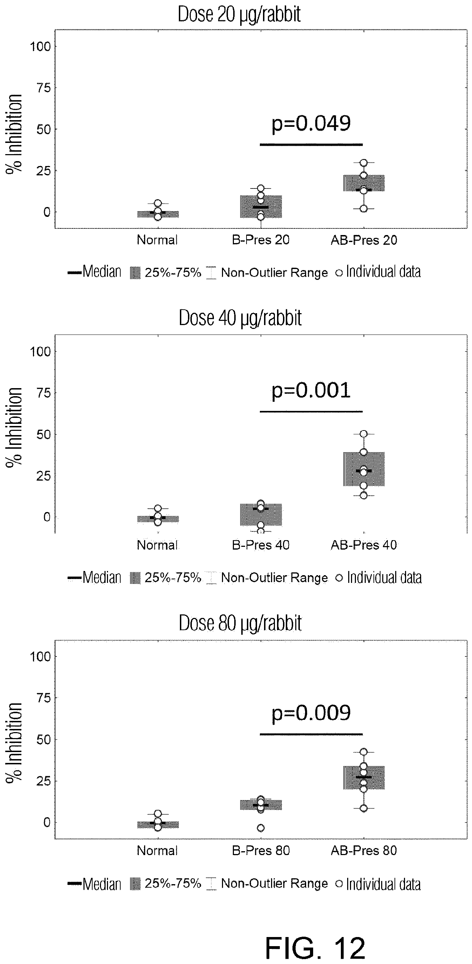

Fig. 12. shows the inhibition of IgE binding to Mal d 1 allergen, using sera from rabbits treated with AIT with various doses of PreS proteins.

The percentage inhibition of IgE binding to rMal d 1 was determined for sera from 10 patients with birch pollen allergy. Preincubation of Bet v 1 with PBS was taken as 0% inhibition. Data are plotted with 50% of the values within the rectangular box; horizontal lines denote median values. The shown significant differences in inhibition were calculated using Student's t-test.

x - statistically significant compared with sera from rabbits immunized with "Allergovit".

**DETAILED DESCRIPTION OF THE INVENTION**

Two vaccines based on PreS protein were additionally developed in order to study whether the introduction of peptides B-PreS derived from allergen Mal d 1 into a vaccine can provide an advantage for inducing protective IgG antibodies to apple allergen. The action of vaccines comprising Mal d 1-derived peptides (A-PreS) and peptides derived from Bet v 1 and Mal d 1 (AB-PreS) were compared with that of a B-PreS-based vaccine and several other commercial vaccines based on birch pollen allergen extracts. The vaccines were also evaluated for their ability to induce IgG antibodies inhibiting the binding of patients' IgE antibodies to Bet v 1 and Mal d 1 allergens.

**EXAMPLE 1. Design of vaccine constructs.**

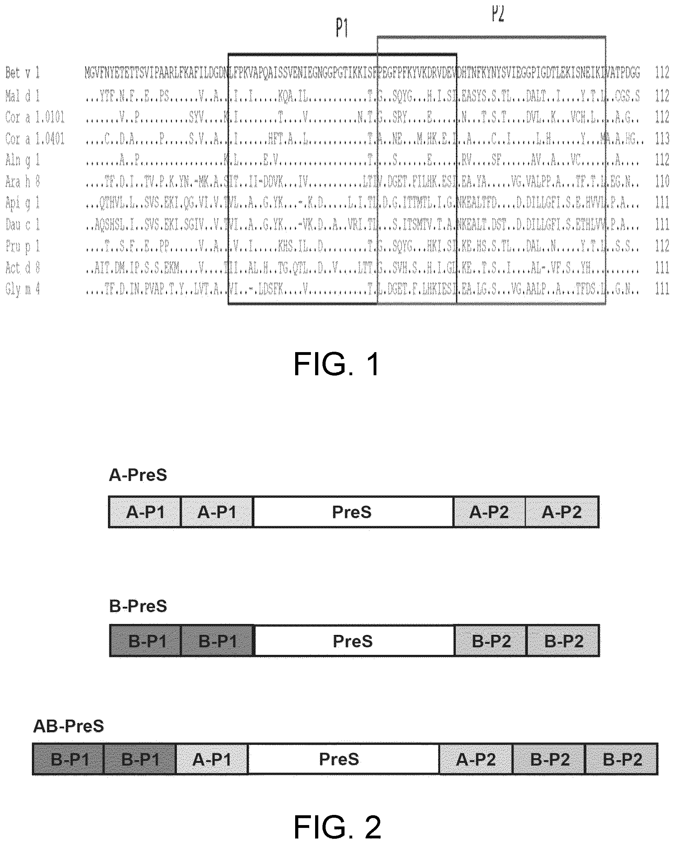

Fig. 1 shows a multiple alignment of the sequence of Bet v 1 with related PR10 allergens from pollen, fruits, and vegetables, from which two peptides of 45 amino acid residues in length (P1, P2) can be derived. These two peptides are derivatives of Bet v 1 from a previously described recombinant PreS protein-based vaccine that can more effectively induce IgG antibodies inhibiting IgE from patients with birch pollen allergy than a recombinant wild-type allergen Bet v 1 [10].

Three recombinant constructs were designed based on PreS protein. B-PreS (Fig. 2) was identical to previously described construct 2PAPB-PreS which was found to activate IgG antibodies capable of inhibiting IgE from patients with birch pollen allergy more effectively than a recombinant wild-type Bet v 1 [10]. Also, two additional constructs were prepared, one of which was similar to construct B-PreS but comprised Mal d 1-derived peptides, instead of Bet v 1-derived peptides from (Fig. 2). Third construct AB-PreS comprised, in addition to Bet v 1-derived peptides, a copy of two Mal d 1-derivatived peptides, A-P1 and A-P2, inserted into B-PreS between the birch allergen peptides and the PreS sequence (Fig. 2) .

The constructs for the expression of recombinant antigens were designed as follows: DNA encoding His-tagged fusion proteins (Fig. 3) consisting of protein PreS (SEQ I D NO: 7) fused with peptides derived from Bet v 1 or Mal d 1, or fused with peptides of Bet v 1 or Mal d 1 derivatives (Mal d 1: A-P1 (30-74) : LIPKIAPQAIKQAEILEGNGGPGTIKKITFGEGSQYGYVKHRIDS (SEQ ID NO: 3), A-P2 (60-104): GEGSQYGYVKHRIDSIDEASYSYSYTLIEGDALTDTIEKISYETK (SEQ ID NO: 4) Bet v 1: B-P1 (aa 30-74): LFPKVAPQAISSVENIEGNGGPGTIKKISFPEGFPFKYVKDRVDE (SEQ ID NO: 5), B-P2 (aa 60-104): PEGFPFKYVKDRVDEVDHTNFKYNYSVIEGGPIGDTLEKISNEIK (SEQ ID NO: 6)) were optimized for expression in *Escherichia coli,* synthesized (construct Mal d 1-PreS A-P2-PreS; construct Bet v 1/construct Mal d 1-PreS: 2B-P1/B-P2+1A-P1/A-P2-PreS-PreS) (ATG: Biosynthetics GmBH, Merzhausen, Germany) (Fig. 2), and inserted at the NdeI/XhoI restriction sites of pET-276 plasmid (Novagen, Darmstadt, Germany). The correctness of the DNA sequence was confirmed by sequencing both strands of the recombinant plasmid (ATG: Biosynthetics GmBH, Merzhausen, Germany).

Recombinant PreS-fusion proteins comprising non-allergenic peptides from IgE binding sites of the main birch pollen allergen Bet v 1 and the main apple allergen Mal d 1 were designed.

Fig. 1 shows the alignment of the Bet v 1 peptide sequences used to develop vaccines, with homologous PR10 allergens from plant foods and pollen. Peptides P1 and P2 derived from Bet v 1 were identical to the previously described construct 2PAPB-PreS that was found to activate IgG antibodies inhibiting IgE from patients with birch pollen allergy more effectively than a recombinant wild-type Bet v 1 [10]. However, this vaccine was not compared with allergen extract-based vaccines for its ability to induce IgG antibodies inhibiting the binding of IgE from patients with allergy to Bet v 1 and Mald 1, the latter of which is one of the most important cross-reactive food allergens for patients with birch pollen allergy. Thus, A-PreS was designed similarly to B-PreS based on homologous peptides A-P1 and A-P2 (Fig. 2). Chimeric construct AB-PreS included peptides from birch pollen and apple allergen, A-P1 and A-P2 (the final protein product had a predicted size of about 50 kPa). The protein sequence of AB-PreS is shown in Fig. 4.

Thus, AB-PreS comprised apple and birch allergen peptides that mimic B-, but not T-cell epitopes of these allergens.

**EXAMPLE 2. Expression, purification, and biochemical characteristic of recombinant polypeptides**

Recombinant PreS-fusion proteins A-PreS, B-PreS and AB-PreS were expressed in *E. coli* strain BL21 (DE3) (Strategene, La Jolla, CA). Transformed cells were grown in Luria-Bertani liquid medium comprising 25 mg/mL kanamycin to an optical density of OD = 0.6-0.8. The protein expression was induced by adding isopropyl-$\beta$-D-thiogalactopiroside to a final concentration of 1 mmol/L, cells were cultured for 3 hours at a temperature of 37°C, followed by centrifugation at 4000 rpm for 20 minutes.

The resulting bacterial pellets were resuspended in 8M urea, 10 mM $NaH_2PO_4$ and 10 mM Tris (pH 8), comprising a mixture of protease inhibitors (Sigma, Aldrich) and 5 pg/mL DNase I (Sigma, Aldrich), then homogenized in Ultra Turrax 3 times for 5 minutes (IKA, Stauffen, Germany) . The pellet of homogenized cells was lysed by stirring for 2 hours at 4°C. Cell lysates were purified by centrifugation at 14,000 rpm for 15 minutes at 4°C, after which recombinant proteins were purified by nickel affinity chromatography (Qiagen, Hilden, Germany). Purified fusion proteins were eluted with 8M urea, 100 mM $NaH_2PO_4$ and 10 mM Tris (pH 4.5), dialyzed with 10 mM NaH2PO4 at pH 4.8 for A-PreS, pH 7.8 for AB-PreS, and pH 7.4 for B-PreS, followed by concentration by ultrafiltration (Amicon Ultra 15; Merck KgaA, Darmstadt, Germany) to 0.75-10 mg/mL. The value was determined using a BCA Protein Assay Kit (Thermo Scientific, IL).

Fig. 6 shows a Coomassie blue stained SDS-PAGE of purified recombinant proteins B-PreS, A-PreS, and AB-PreS. The proteins are well purified to about 95% and are in a significant amount for detection, about 10 mg/l of culture, without any signs of decay. Purification was performed according to the protocol described in the section «Methods». The observed molecular weight in SDS-PAGE for the three proteins matched the predicted one, which was calculated according to their amino acid sequence and the observed masses, which we determined using mass spectrometry (Fig. 6). Circular dichroism (CD) analysis showed that the proteins were not folded.

**EXAMPLE 3. Production of a vaccine composition.**

Each of the recombinant purified proteins was dissolved in an isotonic buffer comprising 0.9% sodium chloride and 10 mM sodium phosphate, and an appropriate amount of aluminum hydroxide was added to each protein solution. A mixture comprising equal parts of four resulting suspensions was prepared and aliquoted under sterile conditions in sealed vials. The injectable composition prepared by this method comprised 0.16 mg/mL each of A-PreS, B-PreS, and AB-PreS.

Vaccines with adsorbed aluminum hydroxide comprising A-PreS, B-PreS, and AB-PreS were formulated as described in [11].

The formulation of the vaccine composition per 1 mL of sterile water was as follows:

1. AB-PreS 0.16 mg
2. Al(OH)$_3$ 1.25 mg.
3. $NaH_2PO_4$ 1.2 mg (final concentration 10 mM).
4. NaCl 9 mg (final concentration 0.9%).

This formulation is required to achieve a maximum dose of 80 $\mu$g AB-PreS per individual in a volume of 0.5 mL/individual. The remaining doses were prepared by diluting the original maximum dose with an appropriate volume of buffer (10 mM NaH2PO4 + 0.9% NaCl) .

**EXAMPLE 4. Immunological characteristics of recombinant protein vaccines *in vitro*.**

The ability of PreS-fused proteins to interact with allergen-specific IgE was evaluated by an ELISA test. Plates were coated with recombinant allergens rBet v1 and rMal d1 or PreS-fusion proteins (A-PreS, B-PreS or AB-PreS) in a concentration of 2 ug/mL at 4°C overnight, then washed and blocked overnight with 2% BSA solution. After that, the plate was incubated overnight at 4°C with 1:10 diluted sera from patients with birch pollen allergy (BPA group), patients without birch pollen allergy (BPA-free group), or healthy volunteers ("HC" group). Bound human IgE antibodies were detected with AP-labeled goat anti-human IgE antibodies (Invitrogen, USA) at a dilution of 1:2500. After washing, ABTS Substrate Solution (10 mg/mL) was added. Optical density was measured at 405 nm.

**4.1. Assessment of hypoallergenic properties of recombinant proteins B-PreS, A-PreS and AB-PreS.**

The potential allergenic activity of the three recombinant PreS proteins was tested in IgE-binding experiments and in basophil activation experiments. The first series of experiments included 84 volunteers (Table 2). The IgE-binding capacity of B-PreS, A-PreS and AB-PreS was compared with that of rBet v 1 in an ELISA test, using sera from 70 patients with birch pollen allergy (Fig. 7). It was found that none of the three recombinant proteins showed any reactivity with IgE antibodies from the sera of patients with birch pollen allergy. However, a significant reaction of the sera of these patients was observed with native allergen rBet v 1. In addition, B-PreS, A-PreS and AB-PreS had no detected IgE reactivity with the sera of patients with allergy to other allergens (not birch pollen) (n = 7) and healthy volunteers (n = 7) (Fig. 7).

**4.2. Blood basophil activation test (BAT test).**

The percentage of active basophils in whole blood samples was assessed by an allergenicity test (BAT test) (Beckman Coulter, Fullerton, CA, USA) according to the manufacturer's instructions. The BAT test was performed using two purified recombinant allergens Bet v 1 and Mal d 1 in three concentrations for each allergen, 1 ng/mL, 10 ng/mL, 100 ng/mL, and with equimolar concentrations of protein constructs B-PreS and A-PreS (2.1 ng/mL, 21 ng/mL, 210 ng/mL), and AB-PreS (2.7 ng/mL, 26.6 ng/mL, 266 ng/mL), which corresponded to a molar concentration of 0.05, 0.5 and 5 nM. The positive control was anti-IgE antibodies (0.01 mg/mL), and the negative control was PBS solvent. Controls were prepared for each patient.

Blood samples were collected in polypropylene tubes comprising K3 EDTA (S-Monovette, Sarstedt, Germany) and used in BAT test immediately after collection. 100 $\mu$L whole blood, 20 $\mu$L test allergen/control and 20 $\mu$L tricolor antibodies (CRTH2-FITC, CD203c-PE, CD3-PC7), and 100 $\mu$L activation solution were used. These components were mixed in plastic test tubes and incubated for 15 minutes at 37°C in a water bath. After incubation, the reaction was stopped by adding 100 $\mu$L of stop solution and 2 mL of fixation/lysis solution. After the reaction was stopped, the samples were incubated at room temperature in a dark place for 10 minutes and centrifuged for 5 minutes at 200 g. After removing the supernatant, 3 mL of PBS were added to each tube, and the samples were centrifuged for 5 min at 200 g. Red blood cells were lysed, and leukocytes were fixed by adding 0.5 mL of 0.1% formaldehyde in PBS.

Flow cytometry was performed on a BD FACS Canto II instrument (BD Biosciences, USA). Compensation was set in BD FACSDiva software (BD Biosciences) . The results were analyzed using FlowJo T software version 10.6.2 (Tree Star). The results are presented as a percentage of activated basophils. Activated basophils were identified as CRTH2-pos CD203c-pos CD3-neg. Isotype controls (rat IgG2a, kappa isotype control (Ebr2a), FITC, Bioscience™, mouse IgGI, (PE Cy7), MG112, Thermofisher), negative (PBS) and positive controls (anti-IgE antibodies) were used to gate activated basophils.

Then, we conducted experiments with four patients with birch pollen allergy, using the blood basophil activation test. The data obtained showed that the activation of basophils by rBet v 1 at a concentration of 5 mM was pronounced, while none of the three recombinant PreS proteins induced the activation of these cells (Fig. 8A). rMal d 1 also induced basophil activation at a concentration of 5 mM in two patients with birch pollen allergy (B1, B4) (Fig. 8A). Experiments were also performed on activation of basophils with a positive control (anti-IgE antibodies), while no activation of basophils was observed when only a buffer solution (negative control) was used (Fig. 8B).

**EXAMPLE 5. Inhibition of the binding of IgE antibodies from patients to recombinant allergens rBet v 1 and rMal d 1, using rabbit antibodies.**

Animals

Forty rabbits (New Zealand white rabbits, females) weighing 2.2-2.5 kg were obtained from KrolInfo (Russia). Before the first immunization, 0.5-1.0 mL serum samples were collected from each rabbit and analyzed using ELISA to determine the background level of IgG antibodies to Bet v 1 and Mal d 1. The study included rabbits with a low natural background level of these antibodies.

Vaccination Protocol

Thirty rabbits with low levels of IgG antibodies to Bet v 1 and Mal d 1 were included in the study after being randomized into 15 groups of 2 rabbits each. Each group of rabbits was individually immunized with 3 recombinant vaccines (A-Pres, B-PreS, AB-PreS) at three doses each and with 6 other commercial vaccines based on allergen extracts:

    1. Pollinex Quattro - Bencard 2. Clustoid - Roxall
    3. Alutard SQ-ALK
    4. Purethal - HAL Allergy
    5. Allergovit - Allergopharma
    6. Allergen extract from birch pollen - Birch Pollen Allergen Extract (Microgen), Russia.

Immunization with commercial allergen extract-based vaccines was performed in accordance with the manufacturer's recommendations for patients with allergies. The order of injections and timing of blood sampling are shown in Fig. 5 and Table 1, where additional information is also given about doses administered and the amount of blood collected.

Recombinant vaccines were prepared by adsorbing three doses (20, 40, 80 μg of each of the recombinant proteins (Apple-Pres: A-Pres; Birch-Pres: B-Pres; Birch/Apple-Pres: AB-Pres) onto aluminum hydroxide, as described for the recombinant grass pollen allergy vaccine BM32 also based on PreS protein [11]. Each rabbit received 5 subcutaneous injections with a total injection volume of 0.5 mL, at 4-week intervals (Table 1, Fig. 5).

Serum sampling (approximately 2 mL from each rabbit) was performed on the day of the first immunization and at 4-week intervals as indicated in Table 1. Sera were stored at -20°C until analysis.

Inhibition of IgE binding in patients with allergy to birch pollen rBet v 1 and apple rMal d 1 was determined by competitive inhibition ELISA. The plates were adsorbed with rBet v 1 or rMal d 1 at a concentration of 1 ug/mL and incubated overnight at 4°C. After washing and blocking overnight with 2% BSA, the plates were incubated for 2 hours at 37°C and then for 1 hour at 4°C with 1:10 diluted sera from rabbits that had received AIT treatment. Serum from non-immunized rabbits was used as a control and compared with serum from rabbits 1 month after the end of the AIT course. Rabbit sera were preincubated for 1 h at 56°C to inactivate IgE but retain other antibody isotypes, in particular IgG antibodies. An additional negative control with phosphate-buffered saline (PBS) was also used that was added to plate wells instead of rabbit serum. After washing, the serum from patients with birch pollen allergy was added at a dilution of 1:5 and incubated overnight at 4°C, then bound human IgE antibodies were detected using HRP-conjugated goat antihuman IgE antibodies (Invitrogen, USA) at a dilution of 1:2000. Inhibition of IgE binding to allergens rBet v 1 or rMal d 1 after preincubation with sera from immunized rabbits was calculated as a percentage for each patient sera as follows:

$$\texttt{percent inhibition = 100-(Odi/OD_{PBS} \times 100),}$$

wherein Odi is optical density after preincubation with serum from immunized rabbits, $OD_{PBS}$ is optical density after preincubation with PBS.

Student's t-test (STATISTICA 8.0 StatSoft Inc) was used to compare the degree of inhibition after different immunization courses. A p value $\leq 0.05$ was considered statistically significant.

### EXAMPLE 6. Determination of inhibition of IgE binding to Bet v 1 upon immunization with B-Pres and AB-PreS

### Example 6.1. Comparative analysis of inhibition of IgE binding to Bet v 1 upon immunization with AB-PreS, B-Pres and A-PreS

The degree of inhibition of the binding of IgE antibodies from patients with allergen Bet v 1, using sera from rabbits that had received AIT courses with recombinant vaccines B-PreS, A-PreS and AB-PreS (in three doses of 20 μg, 40 μg or 80 μg) compared with sera from non-immunized rabbits and rabbits are shown in Fig. 10. Mean inhibition of IgE-binding to Bet v 1, using antibodies from rabbits treated with A-Pres was always less than 50%, while the sera from rabbits immunized with B-PreS and AB-PreS resulted in approximately 75% inhibition, moreover, all three doses inhibited IgE binding significantly better than A-PreS (Fig. 9). There were no significant differences in the degree of inhibition of IgE binding by the sera from rabbits received AIT treatment with B-PreS and AB-PreS vaccines (Fig. 9).

Thus, immunization with B-Pres and AB-PreS induces antibodies that inhibit IgE binding to Bet v 1 significantly stronger than antibodies induced by A-PreS.

### Example 6.2. Comparative analysis of IgE binding to Bet v 1 upon immunization with AB-PreS, B-Pres and commercial vaccines based on birch allergen extracts

Fig. 10 presents data demonstrating that antibodies induced in rabbits with all vaccines (except Purethal) significantly inhibited the binding of IgE antibodies from patients with allergen Bet v 1. The highest degree of inhibition of IgE binding was obtained for rabbit antibodies induced by an allergen extract-based vaccine Allergovit.

It should be noted that recombinant vaccines B-PreS and AB-PreS induce a more significant protective effect compared to commercial Allergovit (Fig. 10). Thus, immunotherapeutic vaccines based on B-PreS and AB-PreS are superior to all existing commercial allergen extract-based vaccines in terms of activation of antibodies blocking the binding of patients' IgE antibodies to allergen Bet v 1.

For most commercial allergen extract-based vaccines, more injections were used than for recombinant PreS protein-based vaccines (Allergovit: 7 injections; Alutard: 15 injections; Birch pollen allergen extract (Microgen): 30 injections; Purethal: 7 injections) (Fig. 5, Table 1). A comparable number of injections were made only for vaccines Pollinex and Clustoid, four and five, respectively (Fig. 5, Table 1). However, the antibodies activated by the last two vaccines only slightly inhibited the binding of patients' IgE antibodies to birch pollen allergen Bet v 1 (Fig. 10) .

Regarding the administration schedule of the commercial vaccines being tested, it should be noted that vaccines Pollinex, Clustoid, Purethal and Microgen's birch pollen allergen extract are to be considered as a pre-season treatment, which means that before the birch flowering season, the same number of injections should be given every year. In summary, a full course of treatment with vaccine Allergovit will require 14 injections over 2 years. Vaccine Alutard can also be administered every year as a pre-season treatment, which includes 30 injections over a two-year treatment period. In addition, monthly injections are also possible, which will also require 15 injections, with 9 of them being administered in the first year of treatment, and 12 injections in an additional year of treatment.

An even greater difficulty is the treatment with sublingual immunotherapy (SLIT), which requires daily administration of the allergen extract, which is one of the reasons for low demand for such therapy. In addition, SLIT induces very low levels of allergen-specific IgG antibodies, and it is unclear whether after such therapy, IgG antibodies are activated enough to block IgE binding in patients with allergy.

In contrast to the allergen extract-based vaccines described above, the treatment regimen for recombinant PreS protein-based vaccines will be a pre-season course of 3-5 injections followed by 1 or 2 pre-season injections in the next year. Thus, the maximum course will be 7 injections for two years of treatment. The suggestion that only 1 or 2 additional injections are needed to increase the level of allergen-specific IgG antibodies after the base course of immunization is supported by the results obtained in clinical trials of the grass pollen allergy vaccine BM32 developed based on PreS protein. The clinical trial showed that one injection is enough to restore allergen-specific IgG antibodies to the levels obtained one month after the main course of immunization [12, 13]. Thus, the treatment with vaccines based on recombinant PreS proteins will be much more convenient than any currently existing form of AIT.

Immunization with B-Pres and AB-PreS induces protective antibodies that inhibit IgE binding to allergen Bet v 1 significantly more strongly than antibodies induced by commercial allergen extract-based vaccines.

**Example 6.3. Comparative analysis of IgE binding to apple allergen Mal d 1 upon immunization with AB-PreS and B-Pres**

Immunizations with two recombinant PreS-based vaccines, B-Pres and AB-Pres, induced similar levels of protective antibodies that inhibited IgE binding to allergen Bet v 1 (Fig. 11). Both vaccines were more effective in terms of activation of antibodies blocking IgE binding to Bet v 1 than the other tested vaccines (Figs 9 and 11). Therefore, B-PreS and AB-PreS were compared for their ability to induce protective antibodies blocking IgE binding to one of the most important cross-reactive food allergens, apple allergen Mal d 1. As can be seen in Fig.12, after immunization with tree doses (20 pg, 40 pg, 80 pg) of AB-PreS, the inhibition of IgE binding to Mal d 1 was significantly more pronounced than the inhibition induced by the same doses of B-PreS. As a result, the recombinant PreS protein-based vaccine AB-PreS against birch pollen allergy induces a higher level of projective antibodies blocking IgE binding to Bet v 1 and Mal d 1 compared to the other tested vaccines.

Antibodies induced by immunization with AB-Pres are significantly better at inhibiting IgE binding to apple allergen Mal d 1 than those induced by B-Pres.

Thus, the produced recombinant polypeptide AB-PreS (SEQ ID NO: 2) that comprises epitopes necessary to activate protective antibodies to Bet v 1 and Mal d 1 in a single protein allows the production of a single-component vaccine, instead of using vaccines comprising two or more derived allergens.

The resulting recombinant polypeptide AB-PreS, which comprises peptides from apple and birch allergens fused with PreS protein, completely lacks IgE reactivity, that indicates a greater safety of this protein. In addition, AB-PreS comprises apple and birch allergen peptides that mimic B-, but not T-cell epitopes of these allergens. Elimination of T-cell epitopes from the AB-PreS composition makes it possible to achieve a more significant safety of the construct due to the exclusion of the possibility of activation of T-cells producing pro-inflammatory factors.

This construct of polypeptide AB-PreS elicits an increased immune response against allergenic molecules and induces blocking IgG antibodies to birch pollen allergen Bet v 1 and to apple allergen Mal d 1. It inhibits IgE binding to Mal d 1 significantly stronger than polypeptide B-PreS at the same doses. As a result, the birch pollen allergy vaccine based on polypeptide AB-PreS induces a higher level of projective antibodies blocking IgE binding to Bet v 1 and Mal d 1, compared to other commercial vaccines.

**REFERENCES**

1. Dorofeeva Y. et al. Past, presence, and future of allergen immunotherapy vaccines // Allergy: European Journal of Allergyand Clinical Immunology. 2020.

2. Eckl-Dorna J. et al. Allergen-specific antibodies regulate secondary allergen-specific immune responses // Frontiers in Immunology. Frontiers Media S.A., 2019. Vol. 9, No 3131. P. 1-15.

3. van Neerven R.J. et al. Blocking antibodies induced by specific allergy vaccination prevent the activation of CD4+ T cells by inhibiting serum-IgE-facilitated allergen presentation. // J. Immunol. 1999.

4. Niederberger V. et al. Vaccination with genetically engineered allergens prevents progression of allergic disease / Proc. Natl. Acad. Sci. U. S. A. 2004. Vol. 101, No 2. P. 14677-14682.

5. Elisyutina O. et al. Bet v 1-specific IgE levels and PR-10 reactivity discriminate silent sensitization from phenotypes of birch allergy / Allergy: European Journal of Allergy and Clinical Immunology. 2019.

6. Gadermaier E. et al. Analysis of the antibody responses induced by subcutaneous injection immunotherapy with birch and Fagales pollen extracts adsorbed onto aluminum hydroxide // Int. Arch. Allergy Immunol. 2010. Vol. 151, No .1 P. 17-27.

7. Bucher X. et al. Effect of tree pollen specific, subcutaneous immunotherapy on the oral allergy syndrome to apple and hazelnut // Allergy Eur. J. Allergy Clin. Immunol. 2004.

8. Valenta R., Campana R., Niederberger V. Recombinant allergy vaccines based on allergen-derived B cell epitopes / Immunology Letters. Elsevier B.V., 2017. Vol. 189. P. 19-26. 9. Valenta R. et al. Vaccine development for allergen-specific immunotherapy based on recombinant allergens and synthetic allergen peptides: Lessons from the past and novel mechanisms of action for the future / J. Allergy Clin. Immunol. Mosby Inc., 2016. Vol. 137, No 2. P. 351-357.

10. Marth K. et al. A nonallergenic birch pollen allergy vaccine consisting of hepatitis PreS-fused Bet v 1 peptides focuses blocking IgG toward IgE epitopes and shifts immune responses to a tolerogenic and ThI phenotype // J. Immunol. The American Association of Immunologists, 2013. Vol. 190, No 7. P. 3068-3078.

11. Zieglmayer P. et al. Mechanisms, safety, and efficacy of a B cell epitope-based vaccine for immunotherapy of grass pollen allergy / BioMedicine. Elsevier B.V., 2016. Vol. 1. P. 43-57.

12. Niederberger V. et al. Safety and efficacy of immunotherapy with the recombinant B-cell epitope-based grass pollen vaccine BM32 /I.J. Allergy Clin. Immunol. Mosby Inc., 2018. Vol. 142, No 2. P. 497-509.

13. Eckl-Dorna J. et al. Two years of treatment with the recombinant grass pollen allergy vaccine BM32 induces a continuously increasing allergen-specific IgG4 response I/ BioMedicine. 2019. Vol. 50. P. 421-432.

Table 1. Immunization schedule and doses

| Group # | Vaccine | Number of rabbits | Dose, μg/ rabbit | Volume, mL/ rabbit | Subcutaneous administration Immunization schedule | Sampling schedule |
|---|---|---|---|---|---|---|
| 1 | Apple-PreS A-PreS | 2 | 20 | 0.5 | 1. Immunization: Day 1<br>2. Immunization: Day 28<br>3. Immunization: Day 56<br>4. Immunization: Day 84<br>5. Immunization: Day 112 | 1. Day 1 (serum before immunization) (2 mL) – before the first injection<br>2. Day 28 (2 mL)<br>3. Day 56 (2 mL)<br>4. Day 84 (2 mL)<br>5. Day 112 (2 mL)<br>6. Day 140 (last sampling day) (40-90 mL) |
| 2 | Apple-PreS A-PreS | 2 | 40 | 0.5 | 1. Immunization: Day 1<br>2. Immunization: Day 28<br>3. Immunization: Day 56<br>4. Immunization: Day 84<br>5. Immunization: Day 112 | 1. Day 1 (serum before immunization) (2 mL) – before the first injection<br>2. Day 28 (2 mL)<br>3. Day 56 (2 mL)<br>4. Day 84 (2 mL)<br>5. Day 112 (2 mL)<br>6. Day 140 (last sampling day) (40-90 mL) |
| 3 | Apple-PreS A-PreS | 2 | 80 | 0.5 | 1. Immunization: Day 1<br>2. Immunization: Day 28<br>3. Immunization: Day 56<br>4. Immunization: Day 84<br>5. Immunization: Day 112 | 1. Day 1 (serum before immunization) (2 mL) – before the first injection<br>2. Day 28 (2 mL)<br>3. Day 56 (2 mL)<br>4. Day 84 (2 mL)<br>5. Day 112 (2 mL)<br>6. Day 140 (last sampling day) (40-90 mL) |
| 4 | Birch-PreS B-PreS | 2 | 20 | 0.5 | 1. Immunization: Day 1<br>2. Immunization: Day 28<br>3. Immunization: Day 56<br>4. Immunization: Day 84<br>5. Immunization: Day 112 | 1. Day 1 (serum before immunization) (2 mL) – before the first injection<br>2. Day 28 (2 mL)<br>3. Day 56 (2 mL)<br>4. Day 84 (2 mL)<br>5. Day 112 (2 mL)<br>6. Day 140 (last sampling day) (40-90 mL) |
| 5 | Birch-PreS B-PreS | 2 | 40 | 0.5 | 1. Immunization: Day 1<br>2. Immunization: Day 28<br>3. Immunization: Day 56<br>4. Immunization: Day 84<br>5. Immunization: Day 112 | 1. Day 1 (serum before immunization) (2 mL) – before the first injection |

| | | | | | | |
|---|---|---|---|---|---|---|
| | | | | | | 2. Day 28 (2 mL)<br>3. Day 56 (2 mL)<br>4. Day 84 (2 mL)<br>5. Day 112 (2 mL)<br>6. Day 140 (last sampling day) (40-90 mL) |
| 6 | Birch-PreS<br>B-PreS | 2 | 80 | 0.5 | 1. Immunization: Day 1<br>2. Immunization: Day 28<br>3. Immunization: Day 56<br>4. Immunization: Day 84<br>5. Immunization: Day 112 | 1. Day 1 (serum before immunization) (2 mL) - before the first injection<br>2. Day 28 (2 mL)<br>3. Day 56 (2 mL)<br>4. Day 84 (2 mL)<br>5. Day 112 (2 mL)<br>6. Day 140 (last sampling day) (40-90 mL) |
| 7 | Birch/Apple-PreS<br>AB-PreS | 2 | 20 | 0.5 | 1. Immunization: Day 1<br>2. Immunization: Day 28<br>3. Immunization: Day 56<br>4. Immunization: Day 84<br>5. Immunization: Day 112 | 1. Day 1 (serum before immunization) (2 mL) - before the first injection<br>2. Day 28 (2 mL)<br>3. Day 56 (2 mL)<br>4. Day 84 (2 mL)<br>5. Day 112 (2 mL)<br>6. Day 140 (last sampling day) (40-90 mL) |
| 8 | Birch/Apple-PreS<br>AB-PreS | 2 | 40 | 0.5 | 1. Immunization: Day 1<br>2. Immunization: Day 28<br>3. Immunization: Day 56<br>4. Immunization: Day 84<br>5. Immunization: Day 112 | 1. Day 1 (serum before immunization) (2 mL) - before the first injection<br>2. Day 28 (2 mL)<br>3. Day 56 (2 mL)<br>4. Day 84 (2 mL)<br>5. Day 112 (2 mL)<br>6. Day 140 (last sampling day) (40-90 mL) |
| 9 | Birch/Apple-PreS<br>AB-PreS | 2 | 80 | 0.5 | 1. Immunization: Day 1<br>2. Immunization: Day 28<br>3. Immunization: Day 56<br>4. Immunization: Day 84<br>5. Immunization: Day 112 | 1. Day 1 (serum before immunization) (2 mL) - before the first injection<br>2. Day 28 (2 mL)<br>3. Day 56 (2 mL)<br>4. Day 84 (2 mL)<br>5. Day 112 (2 mL)<br>6. Day 140 (last sampling day) (40-90 mL) |
| 10 | Pollinex Quattro - Bencard | 2 | | 1 | 1. Immunization: Day 1 300 U (1 mL) from the 300 U/mL flack (green)<br>2. Immunization: Day 14 800 U (1 mL) from the 800 U/mL flack (yellow) | 1. Day 1 (serum before immunization) (2 mL) - before the first injection<br>2. Day 28 (2 mL) |

| | | | | | 3. Immunization: Day 28 2000 U (1 mL) from the 2000 U/mL flack (red)<br>4. Immunization: Day 56 2000 U (1 mL) from the 2000U/mL flack (red) | 3. Day 56 (2 mL)<br>4. Day 84 (last sampling day) (40-90 mL) |
|---|---|---|---|---|---|---|
| 11 | Clustoid – Roxall | 2 | | 0.2 – 0.5 | 1. Immunization: Day 1 One part of 2000 TU (0.2 mL) from the 10000 TU/mL flack (Fx)<br>2. Immunization: Day 1 Second part of 5000 TU in 15 min (0.5 mL) from the 10000 TU/mL flack (Fx)<br>3. Immunization: Day 7 5000 TU (0.5 mL) from the 10000 TU/mL flack (Fx)<br>4. Immunization: Day 14 5000 TU (0.5 mL) from the 10000 TU/mL flack (Fx)<br>5. Immunization: Day 21 5000 TU (0.5 mL) from the 10000 TU/mL flack (Fx) | 1. Day 1 (serum before immunization) (2 mL) – before the first injection<br>2. Day 28 (2 mL)<br>3. Day 49 (last sampling day) (40-90 mL) |
| 12 | Alutard SQ-ALK | 2 | | 0.1 – 1 | 1. Immunization: Day 1 20 SQ-E (0.2 mL) from the 100 SQ-E/mL flack<br>2. Immunization: Day 4 40 SQ-E (0.4 mL) from the 100 SQ-E/mL flack<br>3. Immunization: Day 7 80 SQ-E (0.8 mL) from the 100 SQ-E/mL flack<br>4. Immunization: Day 10 200 SQ-E (0.2 mL) from the 1000 SQ-E/mL flack<br>5. Immunization: Day 13 400 SQ-E (0.4 mL) from the 1000 SQ-E/mL flack<br>6. Immunization: Day 16 800 SQ-E (0.8 mL) from the 1000 SQ-E/mL flack<br>7. Immunization: Day 19 2000 SQ-E (0.2 mL) from the 10000 SQ-E/mL flack<br>8. Immunization: Day 22 4000 SQ-E (0.4 mL) from the 10000 SQ-E/mL flack<br>9. Immunization: Day 25 8000 SQ-E (0.8 mL) from the 10000 SQ-E/mL flack<br>10. Immunization: Day 35 10000 SQ-E (0.1 mL) from the 100000 SQ-E/mL flack<br>11. Immunization: Day 49 20000 SQ-E (0.2 mL) from the 100000 SQ-E/mL flack<br>12. Immunization: Day 63 40000 SQ-E (0.4 mL) from the 100000 SQ-E/mL flack<br>13. Immunization: Day 77 60000 SQ-E (0.1 mL) from the | 1. Day 1 (serum before immunization) (2 mL) – before the first injection<br>2. Day 28 (2 mL)<br>3. Day 56 (2 mL)<br>4. Day 84 (2 mL)<br>5. Day 112 (2 mL)<br>6. Day 133 (last sampling day) (40-90 mL) |

| | | | | | 100000 SQ-E/mL flack<br>14. Immunization: Day 91 80000 SQ-E (0.8 mL) from the 100000 SQ-E/mL flack<br>15. Immunization: Day 105 100000 SQ-E (1 mL) from the 100000 SQ-E/mL flack | |
|---|---|---|---|---|---|---|
| 13 | Purethal – HAL Allergy | 2 | | 0.1 – 0.5 | 1. Immunization: Day 1 1000 TU (0.05 mL) from the 20000 AUM/mL flack<br>2. Immunization: Day 7 2000 TU (0.1 mL) from the 20000 AUM/mL flack<br>3. Immunization: Day 14 4000 TU (0.2 mL) from the 200000 AUM/mL flack<br>4. Immunization: Day 21 6000 TU (0.3 mL) from the 20000 AUM/mL flack<br>5. Immunization: Day 28 8000 TU (0.4 mL) from the 20000 AUM/mL flack<br>6. Immunization: Day 35 10000 TU (0.5 mL) from the 20000 AUM/mL flack | 1. Day 1 (serum before immunization) (2 mL) – before the first injection<br>2. Day 28 (2 mL)<br>3. Day 56 (2 mL)<br>4. Day 63 (last sampling day) (40-90 mL) |
| 14 | Allergovit – Allergopharma | 2 | | 0.1 – 0.8 | 1. Immunization: Day 1 100 TU (0.1 mL) from the 1000 TU/mL flack (A)<br>2. Immunization: Day 7 200 TU (0.2 mL) from the 1000 TU/mL flack (A)<br>3. Immunization: Day 14 400 TU (0.4 mL) from the 10000 TU/mL flack (A)<br>4. Immunization: Day 21 800 TU (0.8 mL) from the 1000 TU/mL flack (A)<br>5. Immunization: Day 35 1500 TU (0.15 mL) from the 10000 TU/mL flack (B)<br>6. Immunization: Day 49 3000 TU (0.3 mL) from the 10000 TU/mL flack (B)<br>7. Immunization: Day 63 6000 TU (0.6 mL) from the 10000 TU/mL flack (B) | 1. Day 1 (serum before immunization) (2 mL) – before the first injection<br>2. Day 28 (2 mL)<br>3. Day 56 (2 mL)<br>4. Day 84 (2 mL)<br>5. Day 91 (last sampling day) (40-90 mL) |
| 15 | Birch pollen allergen extract – Microgen | 2 | | | 1. Immunization: Day 1 0.002 PNU in 0.2 mL<br>2. Immunization: Day 2 0.004 PNU in 0.4 mL<br>3. Immunization: Day 3 0.008 PNU in 0.8 mL<br>4. Immunization: Day 4 0.02 PNU in 0.2 mL<br>5. Immunization: Day 5 0.04 PNU in 0.4 mL<br>6. Immunization: Day 6 0.08 PNU in 0.8 mL<br>7. Immunization: Day 7 0.2 PNU in 0.2 mL<br>8. Immunization: Day 8 | 1. Day 1 (serum before immunization) (2 mL) – before the first injection<br>2. Day 28 (2 mL)<br>3. Day 56 (2 mL) (last sampling day) (40-90 mL) |

| | | | | | | 0.4 PNU in 0.4 mL<br>9. Immunization: Day 9<br>0.8 PNU in 0.8 mL<br>10. Immunization: Day 10<br>2 PNU in 0.2 mL<br>11. Immunization: Day 11<br>4 PNU in 0.4 mL<br>12. Immunization: Day 12<br>8 PNU in 0.8 mL<br>13. Immunization: Day 13<br>20 PNU in 0.2 mL<br>14. Immunization: Day 14<br>30 PNU in 0.4 mL<br>15. Immunization: Day 15<br>40 PNU in 0.8 mL<br>16. Immunization: Day 16<br>50 PNU in 0.5 mL<br>17. Immunization: Day 17<br>60 PNU in 0.6 mL<br>18. Immunization: Day 18<br>70 PNU in 0.7 mL<br>19. Immunization: Day 19<br>80 PNU in 0.8 mL<br>20. Immunization: Day 20<br>90 PNU in 0.9 mL<br>21. Immunization: Day 21<br>100 PNU in 0.1 mL<br>22. Immunization: Day 22<br>200 PNU in 0.2 mL<br>23. Immunization: Day 23<br>300 PNU in 0.3 mL<br>24. Immunization: Day 24<br>400 PNU in 0.4 mL<br>25. Immunization: Day 25<br>500 PNU in 0.5 mL<br>26. Immunization: Day 26<br>600 PNU in 0.6 mL<br>27. Immunization: Day 27<br>700 PNU in 0.7 mL<br>28. Immunization: Day 28<br>800 PNU in 0.8 mL<br>29. Immunization: Day 29<br>900 PNU in 0.9 mL<br>30. Immunization: Day 30<br>1000 PNU in 1.0 mL | |
|---|---|---|---|---|---|---|---|

Table 2. Volunteer groups included in the study on evaluation of the ability of B-PreS, A-PreS and AB-PreS to bind to IgE antibodies.

| No. | Group | Description of volunteers | Number |
|---|---|---|---|
| 1 | BPA | Patients with birch pollen allergy | 70 |
| 2 | Non-BPA | Allergic patients but without birch pollen allergy | 7 |
| 3 | HVs | Healthy volunteers (without allergy) | 7 |
| | Total | | 84 |

Serum samples were collected from patients with birch pollen allergy (BPA), from allergic patients but without birch pollen allergy (non-BPA), or serum samples from non-allergic patients - healthy volunteers (HVs).

# Перечень последовательностей

<110> ФГБУ "ГНЦ Институт иммунологии" ФМБА России

<120> «Рекомбинантный полипептид на основе аллергена пыльцы березы и аллергена яблока в качестве вакцины от аллергии»

<160> NUMBER OF SEQ ID NOS: 7

<210> SEQ ID NO 1

<211> 1353

<212> DNA

<213> artificial

<400> SEQUENCE 1:

ATGCTGTTCCCGAAAGTTGCACCGCAGGCGATCTCCAGCGTCGAGAATATCGA
AGGCAACGGGGGGCCGGGCACCATCAAAAAAATCAGCTTTCCCGAGGGTTTCCCTT
TTAAATACGTTAAAGACCGCGTGGACGAATTGTTTCCAAAAGTGGCGCCACAAGCC
ATCAGTAGTGTCGAAAACATTGAGGGGAACGGGGGCCCTGGTACCATCAAAAAAAT
CTCCTTCCCTGAAGGATTCCCATTCAAATACGTCAAAGATCGTGTAGATGAACTGAT
TCCCAAAATCGCGCCGCAAGCTATCAAGCAAGCAGAAATCCTTGAAGGCAACGGCG
GCCCGGGGACTATCAAAAAAATTACGTTCGGTGAAGGTTCGCAATATGGTTACGTG
AAACATCGTATTGACTCAGGCGGCTGGTCGAGCAAACCTCGTAAAGGTATGGGCAC
CAATCTGTCCGTGCCAAACCCACTGGGCTTCTTTCCAGATCATCAGTTAGACCCGGC
GTTCGGCGCAAACTCAAATAATCCGGACTGGGACTTTAACCCGATTAAAGACCATTG
GCCGGCTGCGAATCAAGTGGGCGTTGGTGCGTTTGGCCCGGGGCTGACCCCGCCGC
ATGGAGGCATCCTGGGCTGGTCGCCGCAAGCTCAAGGTATCTTGACTACCGTTTCCA
CCATTCCCCCGCCGGCCTCAACCAATCGTCAATCTGGCCGTCAACCGACCCCGATCA
GCCCACCCTTGCGCGATAGCCACCCCCAAGCGATGCAGTGGAACAGCACTGCGTTC
CATCAGGCTCTGCAAGATCCACGCGTGCGTGGCTTGTATTTTCCAGCCGGTGGTAGT
TCGTCGGGTACGGTGAACCCGGCCCCAAACATTGCCTCCCACATTAGCTCTATCTCG
GCGCGTACCGGTGACCCGGTTACCAACGGTGAGGGGTCCCAGTATGGCTATGTGAA
ACACCGCATCGATTCAATCGACGAAGCATCGTACTCGTACAGCTACACATTGATCGA
GGGCGATGCACTTACAGATACCATTGAAAAATTTCGTATGAAACCAAACCGGAGG
GGTTCCCCTTTAAATACGTCAAAGATCGCGTGGACGAAGTCGACCATACCAATTTCA
AGTATAACTATTCGGTGATTGAAGGTGGCCCGATCGGCGATACTCTCGAAAAAATTA
GTAATGAAATTAAGCCTGAAGGCTTTCCGTTCAAATACGTTAAGGACCGCGTGGAC
GAAGTTGATCATACCAACTTCAAATATAATTACTCAGTTATTGAAGGTGGTCCGATC

GGGGACACCCTGGAAAAAATCTCGAACGAAATTAAACATCATCATCATCACCACTA
A

<210> SEQ ID NO 2

<211> 443

<212> AA (amino acid)

<213> artificial

<400> SEQUENCE 2:

LFPKVAPQAISSVENIEGNGGPGTIKKISFPEGFPFKYVKDRVDELFPKVAPQAISSV
ENIEGNGGPGTIKKISFPEGFPFKYVKDRVDELIPKIAPQAIKQAEILEGNGGPGTIKKITFG
EGSQYGYVKHRIDSGGWSSKPRKGMGTNLSVPNPLGFFPDHQLDPAFGANSNNPDWDF
NPIKDHWPAANQVGVGAFGPGLTPPHGGILGWSPQAQGILTTVSTIPPPASTNRQSGRQP
TPISPPLRDSHPQAMQWNSTAFHQALQDPRVRGLYFPAGGSSSGTVNPAPNIASHISSISA
RTGDPVTNGEGSQYGYVKHRIDSIDEASYSYSYTLIEGDALTDTIEKISYETKPEGFPFKY
VKDRVDEVDHTNFKYNYSVIEGGPIGDTLEKISNEIKPEGFPFKYVKDRVDEVDHTNFK
YNYSVIEGGPIGDTLEKISNEIK

<210> SEQ ID NO 3

<211> 45

<212> AA (amino acid)

<213> artificial

<400> SEQUENCE 3:

LIPKIAPQAIKQAEILEGNGGPGTIKKITFGEGSQYGYVKHRIDS

<210> SEQ ID NO 4

<211> 45

<212> AA (amino acid)

<213> artificial

<400> SEQUENCE 4:

GEGSQYGYVKHRIDSIDEASYSYSYTLIEGDALTDTIEKISYETK

<210> SEQ ID NO 5

<211> 45

<212> RNA

<213> AA (amino acid)

<400> SEQUENCE 5:

LFPKVAPQAISSVENIEGNGGPGTIKKISFPEGFPFKYVKDRVDE


<210> SEQ ID NO 6

<211> 45

<212> AA (amino acid)

<213> artificial

<400> SEQUENCE 6:

PEGFPFKYVKDRVDEVDHTNFKYNYSVIEGGPIGDTLEKISNEIK


<210> SEQ ID NO 7

<211> 173

<212> AA (amino acid)

<213> artificial

<400> SEQUENCE 7:

GGWSSKPRKGMGTNLSVPNPLGFFPDHQLDPAFGANSNNPDWDFNPIKDHWPAA

NQVGVGAFGPGLTPPHGGILGWSPQAQGILTTVSTIPPPASTNRQSGRQPTPISPPLRDSH

PQAMQWNSTAFHQALQDPRVRGLYFPAGGSSSGTVNPAPNIASHISSISARTGDPVTN


**Claims**

1. A recombinant polypeptide for the treatment and prevention of allergy to birch pollen and apple, comprising peptide fragments of allergens Bet v 1 and Mal d 1, fused with a hepatitis B virus PreS polypeptide, represented by an amino acid sequence of SEO ID NO: 2.

2. The recombinant polypeptide according to claim 1, comprising at least four peptide fragments of wild-type birch pollen allergen Bet v 1 and at least two peptide fragments of wild-type apple allergen Mal d 1.

3. The recombinant polypeptide according to claim 2, wherein the wild-type allergen is selected from the group consisting of major birch pollen allergens, in particular Bet v 1, wherein two peptide fragments have an amino acid sequence of SEQ ID NO: 5 and the other two peptide fragments have an amino acid sequence of SEO ID NO: 6.

4. The recombinant polypeptide according to claim 2, wherein the wild-type allergen is selected from the group consisting of major apple allergens, in particular Mal d 1, having one peptide fragment with an amino acid sequence of SEQ ID NO: 3 and another peptide fragment with an amino acid sequence of SEQ ID NO: 4.

5. A vaccine composition for the treatment or prevention of allergy to birch pollen and apple, comprising an effective amount of the recombinant polypeptide according to claim 1 and a pharmaceutically acceptable excipient.

6. The composition according to claim 5, **characterized by** that the composition further comprises at least one adjuvant and/or preservative.

P1    P2

| Bet v 1 | MGVFNYETETTSVIPAARLFKAFILDGDNLFPKVAPQAISSVENIEGNGGPGTIKKISFPEGFPFKYVKDRVDEVDHTNFKYNYSVIEGGPIGDTLEKISNEIKIVATPDGG | 112 |
| Mal d 1 | ...YTF.N.F..E..PS......V..A...I..I....KQA.IL.............T.S..SQYG...H.I.SI.EASYS.S.TL...DALT..I....Y.T.I..CGS.S | 112 |
| Cor a 1.0101 | ......V..P..........SYV....K.I........T....V........N.T.S..SRY....E.......N...T.S.T....DVL..K...VCH.L...A.G.. | 112 |
| Cor a 1.0401 | ....C..D.A.....P.....S.V.A...I......HFT.A..L............T.A..NE...M.HK.E.I..A....C..I.....L.H......Y...MI.A.JG.. | 113 |
| Aln g 1 | ......A.P..........K.L....E.V...............T...S....E.....RV....SF......AV..A..VC......A.... | 112 |
| Ara h 8 | ....TF.D.I..TV.P.K.YN-.NK.A.SIT..II-DDVK...IV............LTIV.DGET.PILHK.ESI.EA.YA.....VG.VALPP.A...TF.T.I.EG.N.. | 110 |
| Api g 1 | ...QTHVL.L...SVS.EKI.QG.VI.V..IVL..A..G.YK...-.K.D....L.I.TI.D.G.ITTMTL.I.G.NKEALTFD....D.DILLGFI.S.E.HVVL.P.A.... | 111 |
| Dau c 1 | ..AQSHSL.I...SVS.EKI.SGIV..V..IVI..A..G.YK...-VK.D..A..VRI.TI....S.ITSMTV.T.A.NKEALT.DST...D.DILLGFI.S.ETHLVV.P.A.... | 111 |
| Pru p 1 | ....T..S.F..E..PP......V..A...V..I......KHS.IL..D...........T.S..SQYG...HKI.SI.KE.HS.S.TL...DAL..N.....Y.T.I..S.S.. | 112 |
| Act d 8 | ..AIT.DM.IP.S.S.EKM.....V....THI..AL.H...TG.QTL..D..V....LTT.S..SVH.S..H.I.GI.KE..T.S..I.....AL-.VF.S..YH.........  | 111 |
| Gly m 4 | ....TF.D.IN.PVAP.T.Y..LVT.A...VI..-..LDSFK....V............T.L.DGET.F.LHKIESI.EA.IG.S...VG.AALP..A...TFDS.I..G.N.. | 111 |

# FIG. 1

**A-PreS**

| A-P1 | A-P1 | PreS | A-P2 | A-P2 |
|------|------|------|------|------|

**B-PreS**

| B-P1 | B-P1 | PreS | B-P2 | B-P2 |
|------|------|------|------|------|

**AB-PreS**

| B-P1 | B-P1 | A-P1 | PreS | A-P2 | B-P2 | B-P2 |
|------|------|------|------|------|------|------|

# FIG. 2

ATG — Methionine (start)

CTG — Peptide Birch-No1-Copy-1

TTC — Peptide Birch-No1-Copy-2

CTG — Peptide Apple-No1

GGC — PreS protein

GGT — Peptide Apple-No2

CCG — Peptide Birch-No2-Copy-1

CCT — Peptide Birch-No2-Copy-2

CAT — 6x His terminus

TAA — Stop codon

NdeI

CATATGCTGTTCCCGAAAGTTGCACCGCAGGCGATCTCCAGCG
TCGAGAATATCGAAGGCAACGGGGGGGCCGGGCACCATCAAAA
AAATCAGCTTTCCCGAGGGTTTCCCTTTTAAATACGTTAAAGAC
CGCGTGGACGAATTGTTTCCAAAAGTGGCGCCACAAGCCATCA
GTAGTGTCGAAAACATTGAGGGGAACGGGGGCCCTGGTACCA
TCAAAAAAAATCTCCTTCCCTGAAGGATTCCCATTCAAATACGT
CAAAGATCGTGTAGATGAACTGATTCCCAAAATCGCGCCGCAA
GCTATCAAGCAAGCAGAAATCCTTGAAGGCAACGGCGGCCCG
GGGACTATCAAAAAAATTACGTTCGGTGAAGGTTCGCAATATG
GTTACGTGAAACATCGTATTGACTCAGGCGGCTGGTCGAGCAA
ACCTCGTAAAGGTATGGGCACCAATCTGTCCGTGCCAAACCCA
CTGGGCTTCTTTCCAGATCATCAGTTAGACCCGGCGTTCGGCGC
AAACTCAAATAATCCGGACTGGGACTTTAACCCGATTAAAGAC
CATTGGCCGGCTGCGAATCAAGTGGGCGTTGGTGCGTTTGGCC
CGGGGGCTGACCCCGCCGCATGGAGGCATCCTGGGCTGGTCGCC
GCAAGCTCAAGGTATCTTGACTACCGTTTCCACCATTCCCCCGC
CGGCCTCAACCAATCGTCAATCTGGCCGTCAACCGACCCCGAT
CAGCCCACCCTTGCGCGATAGCCACCCCCAAGCGATGCAGTGG
AACAGCACTGCGTTCCATCAGGCTCTGCAAGATCCACGCGTGC
GTGGCTTGTATTTTCCAGCCGGTGGTAGTTCGTCGGGTACGGTG
AACCCGGCCCCAAACATTGCCTCCCACATTAGCTCTATCTCGG
CGCGTACCGGTGACCCGGTTACCAACGGTGAGGGGTCCCAGTA
TGGCTATGTGAAACACCGCATCGATTCAATCGACGAAGCATCG
TACTCGTACAGCTACACATTGATCGAGGGCGATGCACTTACAG
ATACCATTGAAAAAATTTCGTATGAAACCAAACCGGAGGGGTT
CCCCTTTAAATACGTCAAAGATCGCGTGGACGAAGTCGACCAT
ACCAATTTCAAGTATAACTATTCGGTGATTGAAGGTGGCCCGA
TCGGCGATACTCTCGAAAAAATTAGTAATGAAATTAAGCCTGA
AGGCTTTCCGTTCAAATACGTTAAGGACCGCGTGGACGAAGTT
GATCATACCAACTTCAAATATAATTACTCAGTTATTGAAGGTG
GTCCGATCGGGGACACCCTGGAAAAAAATCTCGAACGAAATTA
AACATCATCATCATCACCACTAACTCGAG
                                    XhoI

## FIG. 3

M – Methionine (start)

LFPK – Peptide Birch-No1-Copy-1

LFPK – Peptide Birch-No1-Copy-2

LIPKI – Peptide Apple-No1

GGWS – PreS protein

GEGS - Peptide Apple-No2

PEGF - Peptide Birch-No2-Copy-1

PEGF - Peptide Birch-No2-Copy-2

HHHHHH - 6x His terminus

* - Stop codon

HMLFPKVAPQAISSVENIEGNGGPGTIKKISFPEGFPFKYVKDRVD
ELFPKVAPQAISSVENIEGNGGPGTIKKISFPEGFPFKYVKDRVDE
LIPKIAPQAIKQAEILEGNGGPGTIKKITFGEGSQYGYVKHRIDSG
GWSSKPRKGMGTNLSVPNPLGFFPDHQLDPAFGANSNNPDWDF
NPIKDHWPAANQVGVGAFGPGLTPPHGGILGWSPQAQGILTTVS
TIPPPASTNRQSGRQPTPISPPLRDSHPQAMQWNSTAFHQALQDP
RVRGLYFPAGGSSSGTVNPAPNIASHISSISARTGDPVTNGEGSQY
GYVKHRIDSIDEASYSYSYTLIEGDALTDTIEKISYETKPEGFPFKY
VKDRVDEVDHTNFKYNYSVIEGGPIGDTLEKISNEIKPEGFPFKY
VKDRVDEVDHTNFKYNYSVIEGGPIGDTLEKISNEIKHHHHHH*L
E

# FIG. 4

FIG. 5

FIG. 6

FIG. 7

A  Activation of basophils by using PreS proteins

Б  Positive and negative controls for basophil activation

FIG. 8

## Dose 20 µg/rabbit

Legend: —Median ▓ 25%-75% ⊥ Non-Outlier Range ○ Individual data

## Dose 40 µg/rabbit

Legend: —Median ▓ 25%-75% ⊥ Non-Outlier Range ○ Individual data

## Dose 80 µg/rabbit

Legend: — Median ▓ 25%-75% ⊥ Non-Outlier Range ○ Individual data

FIG. 9

Commercial extract-based vaccines

FIG. 10

Comparison of PreS protein-based vaccines with vaccine Allergovit

—Median ▓ 25%-75% ⊥ Non-Outlier Range ○Individual data

FIG. 11

FIG. 12

## INTERNATIONAL SEARCH REPORT

| International application No. |
| --- |
| PCT/RU 2021/000437 |

**A. CLASSIFICATION OF SUBJECT MATTER**

(see supplemental sheet)

According to International Patent Classification (IPC) or to both national classification and IPC

**B. FIELDS SEARCHED**

Minimum documentation searched (classification system followed by classification symbols)

A61K, C07K, A61P

Documentation searched other than minimum documentation to the extent that such documents are included in the fields searched

Electronic data base consulted during the international search (name of data base and, where practicable, search terms used)

E-Library, Espacenet, PatSearch, PATENTSCOPE, RUPTO, Google, Google Scholar, PubMed, USPTO, ScienceDirect

**C. DOCUMENTS CONSIDERED TO BE RELEVANT**

| Category* | Citation of document, with indication, where appropriate, of the relevant passages | Relevant to claim No. |
| --- | --- | --- |
| A | RU 2013157115 A (BIOMEI AG), 20.07.2015 | 1-6 |
| A | RU 2624030 C2 (LOFARMA S.P.A), 31.07.2012 | 1-6 |
| A | RU 2193413 C2 (NOVARTIS AG), 01.03.1996 | 1-6 |

☐ Further documents are listed in the continuation of Box C.   ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 09 December 2021 (09.12.2021) | 20 January 2022 (20.01.2022) |

| Name and mailing address of the ISA/ RU | Authorized officer |
| --- | --- |
| Facsimile No. | Telephone No. |

Form PCT/ISA/210 (second sheet) (July 1998)

**INTERNATIONAL SEARCH REPORT**

International application No.

**Box No. I      Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

a.  [X]  forming part of the international application as filed:

   [X]  in the form of an Annex C/ST.25 text file.

   [ ]  on paper or in the form of an image file.

b.  [ ]  furnished together with the international application under PCT Rule 13*ter*.1(a) for the purposes of international search only in the form of an Annex C/ST.25 text file.

c.  [ ]  furnished subsequent to the international filing date for the purposes of international search only:

   [ ]  in the form of an Annex C/ST.25 text file (Rule 13*ter*.1(a)).

   [ ]  on paper or in the form of an image file (Rule 13*ter*.1(b) and Administrative Instructions, Section 713).

2.  [ ]  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet (1)) (January 2015)

**INTERNATIONAL SEARCH REPORT**

International application No.

PCT/RU 2021/000437

A.    CLASSIFICATION OF SUBJECT MATTER

*A61K 39/35* (2006.01)
*A61K 39/36* (2006.01)
*A61K 39/29* (2006.01)
*C07K 14/02* (2006.01)
*A61K 38/17* (2006.01)
*A61P 37/08* (2006.01)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03072601 A
- WO 0240676 A
- WO 2013017591 A
- WO 2007140505 A
- WO 2012168487 A

### Non-patent literature cited in the description

- **DOROFEEVA Y. et al.** Past, presence, and future of allergen immunotherapy vaccines. *Allergy: European Journal of Allergyand Clinical Immunology,* 2020
- **ECKL-DORNA J. et al.** Allergen-specific antibodies regulate secondary allergen-specific immune responses. *Frontiers in Immunology. Frontiers Media S.A.,* 2019, vol. 9 (3131), 1-15
- **VAN NEERVEN R.J. et al.** Blocking antibodies induced by specific allergy vaccination prevent the activation of CD4+ T cells by inhibiting serum-IgE-facilitated allergen presentation. *J. Immunol.,* 1999
- **NIEDERBERGER V. et al.** Vaccination with genetically engineered allergens prevents progression of allergic disease. *Proc. Natl. Acad. Sci. U. S. A.,* 2004, vol. 101 (2), 14677-14682
- **ELISYUTINA O. et al.** Bet v 1-specific IgE levels and PR-10 reactivity discriminate silent sensitization from phenotypes of birch allergy. *Allergy: European Journal of Allergy and Clinical Immunology,* 2019
- **GADERMAIER E. et al.** Analysis of the antibody responses induced by subcutaneous injection immunotherapy with birch and Fagales pollen extracts adsorbed onto aluminum hydroxide. *Int. Arch. Allergy Immunol.,* 2010, vol. 151 (1), 17-27
- **BUCHER X. et al.** Effect of tree pollen specific, subcutaneous immunotherapy on the oral allergy syndrome to apple and hazelnut. *Allergy Eur. J. Allergy Clin. Immunol.,* 2004
- Recombinant allergy vaccines based on allergen-derived B cell epitopes. **VALENTA R. ; CAMPANA R. ; NIEDERBERGER V.** Immunology Letters. Elsevier B.V, 2017, vol. 189, 19-26
- Vaccine development for allergen-specific immunotherapy based on recombinant allergens and synthetic allergen peptides: Lessons from the past and novel mechanisms of action for the future. **VALENTA R. et al.** J. Allergy Clin. Immunol. Mosby Inc, 2016, vol. 137, 351-357
- A nonallergenic birch pollen allergy vaccine consisting of hepatitis PreS-fused Bet v 1 peptides focuses blocking IgG toward IgE epitopes and shifts immune responses to a tolerogenic and ThI phenotype. **MARTH K. et al.** J. Immunol. The American Association of Immunologists, 2013, vol. 190, 3068-3078
- Mechanisms, safety, and efficacy of a B cell epitope-based vaccine for immunotherapy of grass pollen allergy. **ZIEGLMAYER P. et al.** BioMedicine. Elsevier B.V, 2016, vol. 1, 43-57
- Safety and efficacy of immunotherapy with the recombinant B-cell epitope-based grass pollen vaccine BM32. **NIEDERBERGER V. et al.** I.J. Allergy Clin. Immunol. Mosby Inc, 2018, vol. 142, 497-509
- **ECKL-DORNA J. et al.** Two years of treatment with the recombinant grass pollen allergy vaccine BM32 induces a continuously increasing allergen-specific IgG4 response I. *BioMedicine,* 2019, vol. 50, 421-432